# EUROPEAN PATENT APPLICATION

(11) **EP 1 704 877 A1**
(43) Date of publication of application: **27.09.2006**
(21) Application number: 05006495.5
(22) Date of filing: 24.03.2005
(51) Int. Cl.: A61L 15/44

(54) **A wound care device comprising a nitric oxide eluting polymer**

(71) Applicant: NOLabs AB, 252 21 Helsingborg (SE)
(72) Inventor: Peters, Tor, 8703 Erlenbach (CH)
(74) Representative: Petri, Stellan

(57) **Abstract**

A device, and a manufacturing process of said device, is provided that allows for treatment of wounds. The device comprises a nitric oxide (NO) eluting polymer arranged to contact the wound area to be treated, such that a therapeutic dose of nitric oxide is eluted from said nitric oxide eluting polymer to said wound area, thus promoting healing of a wound at said wound area.

## Description

### Field of the Invention

This invention pertains in general to the field of a device for wound care, involving the use of nitric oxide (NO). More particularly the invention relates to a device for performing said wound care, and a process for manufacturing of said device, involving the use of nitric oxide (NO).

### Background of the Invention

Wounds, of some sort, are a common occurrence in the daily life. These wounds may vary widely, depending on how they are obtained. Irrespectively, these wounds need to be taken care of in one way or another.

The normal way to take care of such wounds, according to the prior art, is to provide the wound with a wound care, such as plaster, bandage, dressing, wound dressing, pressure bandage etc., depending on the severity and size of the obtained wound.

However, the wound cares according to the prior art do only consider the stoppage of blood flow and prevention of microbial attacks in the inflicted wound. They do not promote healing or present an anti-microbial, anti-inflammatory, or anti-viral, effect in a satisfactory way.

Nitric oxide (NO) is a highly reactive molecule that is involved in many cell functions. In fact, nitric oxide plays a crucial role in the immune system and is utilized as an effector molecule by macrophages to protect itself against a number of pathogens, such as fungi, viruses, bacteria etc., and general microbial invasion. This improvement of healing is partly caused by NO inhibiting the activation or aggregation of blood platelets, and also by NO causing a reduction of inflammatory processes at the site of an implant.

NO is also known to have an anti-pathogenic, especially an anti-viral, effect, and furthermore NO has an anti-cancerous effect, as it is cytotoxic and cytostatic in therapeutic concentrations, i.e. it has among other effects tumoricidal and bacteriocidal effects. NO has for instance cytotoxic effects on human haematological malignant cells from patients with leukaemia or lymphoma, whereby NO may be used as a chemotherapeutic agent for treating such haematological disorders, even when the cells have become resistant to conventional anti-cancer drugs. This anti-pathogenic and anti-tumour effect of NO is taken advantage of by the present invention, without having adverse effects.

However, due to the short half-life of NO, it has hitherto been very hard to treat viral, bacteria, virus, fungi or yeast infections with NO. This is because NO is actually toxic in high concentrations and has negative effects when applied in too large amounts to the body.

NO is actually also a vasodilator, and too large amounts of NO introduced into the body will cause a complete collapse of the circulatory system. On the other hand, NO has a very short half-life of fractions of a second up to a few seconds, once it is released. Hence, administration limitations due to short half-life and toxicity of NO have been limiting factors in the use of NO in the field of anti-pathogenic and anti-cancerous treatment so far.

In recent years research has been directed to polymers with the capability of releasing nitrogen oxide when getting in contact with water. Such polymers are for example polyalkyleneimines, such as L-PEI (Linear PolyEthyleneImine) and B-PEI (Branched PolyEthyleneImine), which polymers have the advantage of being biocompatible with natural products, after the release of nitrogen oxide.

Other example for NO eluting polymers are given in US-5,770,645, wherein polymers derivatized with at least one -NOₓ group per 1200 atomic mass unit of the polymer are disclosed, X being one or two. One example is an S-nitrosylated polymer and is prepared by reacting a polythiolated polymer with a nitrosylating agent under conditions suitable for nitrosylating free thiol groups.

Akron University has developed NO-eluting L-PEI molecule that can be nano-spun onto the surface of medical devices to be permanently implanted in the body, such as implanted grafts, showing significant improvement of the healing process and reduced inflammation when implanting such devices. According to US-6,737,447, a coating for medical devices provides nitric oxide delivery using nanofibers of linear poly(ethylenimine)-diazeniumdiolate. Linear poly(ethylenimine)diazeniumdiolate releases nitric oxide (NO) in a controlled manner to tissues and organs to aid the healing process and to prevent injury to tissues at risk of injury. Electrospun nano-fibers of linear poly(ethylenimine) diazeniumdiolate deliver therapeutic levels of NO to the tissues surrounding a medical device while minimizing the alteration of the properties of the device. A nanofiber coating, because of the small size and large surface area per unit mass of the nanofibers, provides a much larger surface area per unit mass while minimizing changes in other properties of the device.

However, the disclosure is both silent concerning an improvement of present technology in respect of treatment of wounds by the use of NO.

Hence, an improved device for the treatment of wounds, which device presents an improved wound healing and anti-microbial, anti-inflammatory, and anti-viral effect, would be advantageous, and in particular a device allowing for target treatment of wounds would be advantageous.

### Summary of the Invention

Accordingly, the present invention preferably seeks to mitigate, alleviate or eliminate one or more of the above-identified deficiencies in the art and disadvantages singly or in any combination and solves at least the problems mentioned above, by providing a device for said treatment of wounds, a manufacturing method for the latter and a use of nitric oxide according to the appended patent claims.

According to one aspect of the invention, a device is provided that allows for target treatment of wounds. The device comprises a nitric oxide (NO) eluting polymer arranged to contact the area to be treated, such that a therapeutic dose of nitric oxide is eluted from said nitric oxide eluting polymer to said area.

According to another aspect of the invention, a manufacturing process for such a device is provided, wherein the process is a process for forming a device that allows for target treatment of wounds. The process comprises selecting a plurality of nitric oxide eluting polymeric particles, such as nano fibres, fibres, nano particles, or microspeheres, and deploying said nitric oxide eluting particles in a condom/sheath, tape/coating, fibres, nano-particles, or micro-spheres to be comprised in said device. Alternatively the NO eluting particles are admixed to an ointment, gel, hydrogel, foam, or cream.

The present invention has at least the advantage over the prior art that it provides target exposure of a wound to NO, whereby blood perfusion and vasodilatation are increased, simultaneously as an anti-viral, an anti-inflammatory, and an anti-microbial, therapy is achievable.

### Brief Description of the Drawings

These and other aspects, features and advantages of which the invention is capable of will be apparent and elucidated from the following description of embodiments of the present invention, reference being made to the accompanying drawings, in which
Fig. 1 is a schematic illustration of a patch/pad according to the invention,
Fig. 2 is a schematic illustration of a tape or coating according to the invention,
Fig. 3 is a schematic illustration of a condom/sheath according to the invention, and
Fig. 4 is a schematic illustration of a sock according to the invention.

### Description of Embodiments

The following description focuses on embodiments of the present invention applicable to a device, which allows for target treatment of wounds.

With regard to nitric oxide (nitrogen monoxide, NO), its physiological and pharmacological roles have attracted much attention and thus have been studied. NO is synthesized from arginine as the substrate by nitric oxide synthase (NOS). NOS is classified into a constitutive enzyme, cNOS, which is present even in the normal state of a living body and an inducible enzyme, iNOS, which is produced in a large amount in response to a certain stimulus. It is known that, as compared with the concentration of NO produced by cNOS, the concentration of NO produced by iNOS is 2 to 3 orders higher, and that iNOS produces an extremely large amount of NO.

In the case of the generation of a large amount of NO as in the case of the production by iNOS, it is known that NO reacts with active oxygen to attack exogenous microorganisms and cancer cells, but also to cause inflammation and tissue injury. On the other hand, in the case of the generation of a small amount of NO as in the case of the production by cNOS, it is considered that NO takes charge of various protective actions for a living body through cyclic GMP (cGMP), such as vasodilator action, improvement of the blood circulation, anti-platelet-aggregating action, anti-bacterial action, anti-viral action, anti-inflammatory action, anticancer action, acceleration of the absorption at the digestive tract, renal function regulation, neurotransmitting action, erection (reproduction), learning, appetite, and the like. Heretofore, inhibitors of the enzymatic activity of NOS have been examined for the purpose of preventing inflammation and tissue injury, which are considered to be attributable to NO generated in a large amount in a living body. However, the promotion of the enzymatic activity (or expressed amount) of NOS (in particular, cNOS) has not been examined for the purpose of exhibiting various protective actions for a living body by promoting the enzymatic activity of NOS and producing NO appropriately.

In recent years research has been directed to polymers with the capability of releasing nitrogen oxide when getting in contact with water. Such polymers are for example polyalkyleneimines, such as L-PEI (Linear PolyEthyleneImine) and B-PEI (Branched PolyEthyleneImine), which polymers have the advantage of being biocompatible, after the release of nitrogen oxide. Another advantage is that NO is released without any secondary products that could lead to undesired side effects.

The polymers according to the present invention may be manufactured by electro spinning. Electro spinning is a process by which a suspended polymer is charged. At a characteristic voltage a fine jet of polymer releases from the surface in response to the tensile forces generated by interaction by an applied electric field with the electrical charge carried by the jet. This process produces a bundle of polymer fibres, such as nano-fibres. This jet of polymer fibres may be directed to a surface to be treated.

Furthermore, US 6,382,526, US 6,520,425, and US 6,695,992 disclose processes and apparatuses for the production of such polymeric fibres. These techniques are generally based on gas stream spinning, also known within the fiber forming industry as air spinning, of liquids and/or solutions capable of forming fibers.

Other example for NO eluting polymers are given in US-5,770,645, wherein polymers derivatized with at least one -NOX group per 1200 atomic mass unit of the polymer are disclosed, X being one or two. One example is an S-nitrosylated polymer and is prepared by reacting a polythiolated polymer with a nitrosylating agent under conditions suitable for nitrosylating free thiol groups.

Akron University has developed NO-eluting L-PEI molecule that can be nano-spun onto the surface of permanently implanted medical devices, such as implanted grafts, showing significant improvement of the healing process and reduced inflammation when implanting such devices. According to US-6,737,447, a coating for medical devices provides nitric oxide delivery using nanofibers of linear poly(ethylenimine)-diazeniumdiolate. Linear poly(ethylenimine)diazeniumdiolate releases nitric oxide (NO) in a controlled manner.

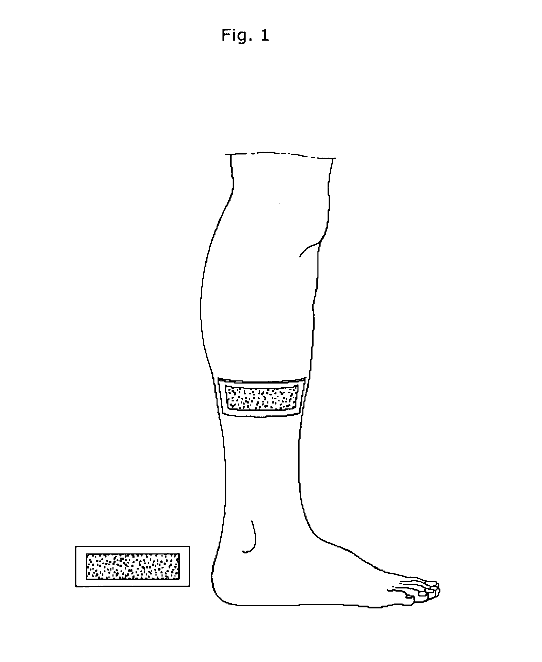

In one embodiment of the device according to the present invention, said device is in form of a patch/pad, according to Fig. 1, which patch/pad is suitable to be applied on the face, arm, hand, thigh, back, stomach, neck, on which body part the wound to be treated is located.

In another embodiment of the present invention this patch/pad is attached by any suitable adhering means, such as materials that adhere to the skin.

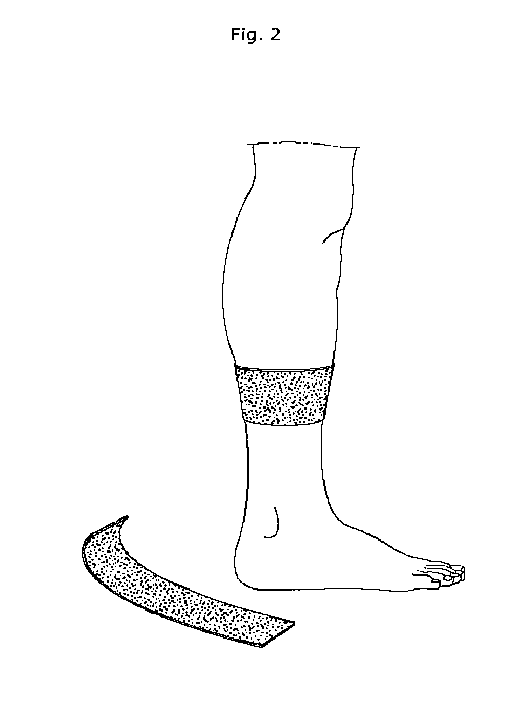

In another embodiment of the device according to the present invention, said device may be manufactured in the form of a polyurethane, or polyethylene, tape or coating, according to Fig. 2. This polyurethane tape or coating may easily be wrapped around, or applied on, the wounded area to be treated. At least the side facing the body may be covered with NO-eluting nano-particles, or micro-spheres, or nano-filament of NO-eluting L-PEI. When these particles or filaments get in contact with the moisture, in form of sweat, on the inside of the tape or coating, the elution of NO starts.

In other embodiments of the invention, the patch/pad or tape/coating may be manufactured by any other suitable material, such as polyvinylacetates, polylacticacids, polyesters, polyamides, polyethers, polyurethanes, polycarbonates, cotton, starch, cellulose, polyhydroxyalkanoates, polyesters, polycaprolactone, polyvinylalcohol, polypropylene, polyacrylonitrile, polystyrene, poly(acrylic acid), protein based plastics, gelatine, biogradable polymers, and other soluble plastics. The NO-eluting polymer may be integrated in, spun together with, or spun on top of, any of these materials in all of the embodiments of the present invention.

The device according to the present invention is applied on the area to be treated, such as any part of the body in need of treatment of at least one wound, such as the face, neck, shoulders, hands, arms, back, chest, stomach, bottom, thigh, genitals, lower leg, and/or foot.

When the patch/pad according to the present invention has been applied, an elution of NO is initiated by adding water in any possible way. This may for example be accomplished by applying a water soaked patch on said patch/pad, or spraying or bathing said patch/pad with water. Often, the moisture/water obtained from the sweat secreted underneath the device according to the present invention, is enough to obtain a satisfactory elution of NO from said NO eluting polymer.

The healing effect of the wound is obtained, as the NO eluting polymer elutes NO on the area to be treated, by an increased blood perfusion and vasodilatation, whereby an increased healing effect of the tissue of interest is achieved.

The increased blood perfusion and vasodilatation may, in another embodiment of the present invention, result in an improved effect when combined with other wound care products, comprising active components. Thus, the synergistic effect from NO and other wound healing, or anti-microbial, anti-inflammatory, or anti-viral, components is within the scope of the present invention.

In another embodiment of the present invention the device is in form of fibres, nano-particles, or micro-spheres of a NO eluting polymer, which fibres, nano-particles, or micro-spheres are be integrated in a gel, cream, or foam, that may either be in a smearing or compressed structure.

These fibres, nano-particles, or micro-spheres, may be formed from the NO-eluting polymers comprised in the present invention, for example polyalkyleneimines, such as L-PEI (Linear PolyEthyleneImine) and B-PEI (Branched PolyEthyleneImine), which polymers have the advantage of being biocompatible, after the release of nitrogen oxide. They may also be encapsulated in any suitable material, such as polyvinylacetates, polylacticacids, starch, cellulose, polyhydroxyalkanoates, polyesters, polycaprolactone, polyvinylalcohol, protein based plastics, gelatine, biogradable polymers, and other soluble plastics.

The fibres, nano-particles, or micro-spheres may also be integrated in a hydrogel, which is mixed directly before use.

This embodiment has the advantage of being able to penetrate pockets and corners in the skin, and/or wound, for closer elution of NO on the area to be treated.

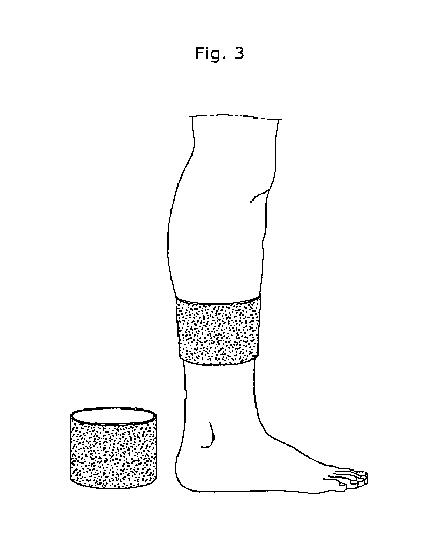

In another embodiment of the invention, according to Fig. 3, the device according to the present invention is in form of a latex or rubber condom/sheath, said condom/sheath being covered with nano-filament of any of the NO-eluting polymers according to above, such as polyalkyleneimines, such as L-PEI (Linear PolyEthyleneImine) and B-PEI (Branched PolyEthyleneImine), which polymers have the advantage of being biocompatible, after the release of nitrogen oxide.

In another embodiment of the present invention the condom/sheath is covered on the inside with nano-filament of L-PEI.

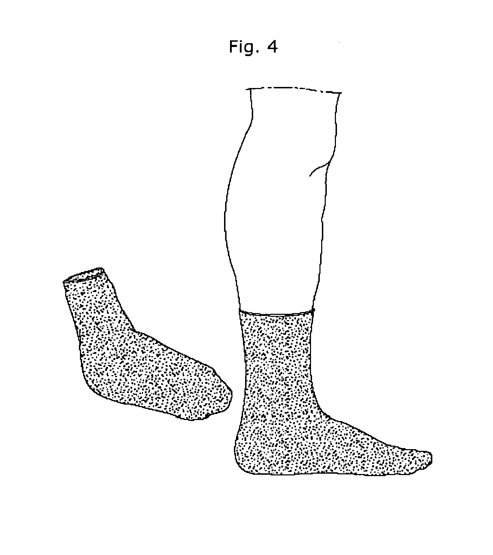

This condom/sheath may be in any suitable size, such as a suitable size for rolling said condom/sheath over the thigh, arm, neck, head, foot etc., to be treated. These sizes may for example vary from small, medium, and large sized condoms/sheaths in accordance with the different sizes, in respect of the different body parts, of persons in the population. The condom/sheath according to the invention may even have a size suitable for covering a foot, such as a sock, according to Fig. 4, or a foot-condom/sheath, or other specific part of the body, to be able to obtain treatment of a wound. According to an embodiment, the condoms/sheaths are coated with NO eluting nano fibres. According to another embodiment the condoms/sheaths are made of, or comprise nanofilaments, e.g. made by electro or gas jet spinning. According to a further embodiment the condoms/sheaths comprises microspheres eluting NO in use. Preferably the three aforementioned embodiments employ L-PEI material loaded with NO. Activation on NO release may be done by e.g. foot sweat, water sprayed onto the condoms/sheaths immediately prior to use, or a water bag configured for releasing water upon activation, e.g. by pushing onto the bag thus bursting (see below).

When the NO-eluting condom/sheath according to certain embodiments of the present invention is treated with or gets in contact with the moisture, in form of secreted sweat, the NO-eluting condom/sheath starts to elute NO to the area to be treated. Alternatively the device is moistured or wettened immediately prior to application or use for controlling or activating the elution of NO.

In another embodiment of the present invention the condom/sheath, sock, patch/pad, or tape/coating, is covered on the inside with NO-eluting nano-particles, or micro-spheres, according to above.

When the nano-particles, or micro-spheres, according to this embodiment, gets in contact with the secreted moisture, in form of sweat, on the inside of the condom/sheath, they start to elute NO on the area to be treated.

In yet another embodiment of the present invention the condom/sheath, sock, patch/pad, or tape/coating, contains a small water bag or sealed water sponge. This water bag or sealed water sponge is used to activate the elution of NO from the NO-eluting nano-particles, or micro-spheres. Persons that not easily sweat may be helped by the use of this embodiment.

In another embodiment the fibres, nano-particles, or micro-spheres, may be integrated in a soluble film that disintegrates on the inside of the condom/sheath, sock, patch/pad, or tape/coating, according to the present invention, in order to elute NO at the area of interest when the soluble film gets in contact with the moisture, in form of sweat or from the water bag or sealed water sponge, on the area to be treated.

When placed on an area to be treated the device according to the present invention provides prevention and treatment of wounds.

In another embodiment of the present invention the device according to the present invention only allows NO-elution in one direction. In this kind of embodiment one side of the device according to the invention, such as condom/sheath, sock, patch/pad, or tape/coating, is non-permeable to NO. This may be accomplished by applying a material on one side of the device according to the invention that is not permeable to NO. Such materials may be chosen from the group comprising common plastics, such as polyethylene, polyurethane etc. This embodiment is also easy to manufacture as the NO eluting polymer, e.g. L-PEI nano fibres may be electro or gas-jet spun onto the surface of the-device according to the invention of e.g. the mentioned plastics, latex, or cotton. In the case of a condom it may be rolled up, or a sheath may be turned outside in after manufacturing to protect the NO eluting polymer during packaging, transport and prior to use from external influences, being e.g. mechanical (abrasion of the polymer), chemical (moisture deactivating the device prior to use) etc.

In yet another embodiment of the present invention the NO-eluting device is acting as a booster for drug eluting patches, e.g. pharmaceuticals, vitamins, nicotin, nitroglycerin, Non-Steroidal Anti.Inflammatory Drugs (NSAID), such as diclofenac, ibuprofen, aspirin, naproxen, COX-2 inhibitors, choline magnesium trisalicylate, diflunisal, salsalate, fenoprofen, flurbiprofen, ketoprofen, oxaprozin, indomethacin, sulindac, tolmetin, meloxicam, piroxicam, meclofenamate, mefenamic acid, nabumetone, etodalac, ketorolac, celecoxib, valdecoxib, and rofecoxib; steroids, such as cortisone, prednisone, methylprednisolone, prednisolone, vitamin D, estrogen, cholestrol, beclomethasone, flunisolide, fluticasone, triamcinolone, desonide, clobetasol, alclometasole, desoximetasone, betamethasone, halcinonide and dexamethasone; pain reliefs, such as motrin, feldene, naprosyn, lidocaine, and prilocaine; and other substances, such as indinavirsulfate, finasteride, aprepitant, montelukast sodium, alendronate sodium, rofecoxib, rizatriptan benzoate, simvastatin, finasteride, ezetimibe, caspofungin acetate, ertapenem sodium, dorzolamide hydrochloride, timolol maleate, losartan potassium, and hydrochlorotiazide; etc. This embodiment presents a device with the advantage of combining two treatments, of significant value, in one treatment.

Hence, such devices may achieve a synergetic effect, when NO is eluted from the devices. NO has a vasodilatory effect on the region where the device having the combination compound actuates. Vasodilated tissue is more susceptible to certain medications and thus more easily treated by the medical preparations and still NO has in addition to that the anti-inflammatory, anti-bacterial etc. effect. Hence, an unexpected surprisingly effective treatment is provided.

In another embodiment according to the present invention NO eluting poymer is applied on devices for ostomy care, such as a ostomy bag, gaskets for said ostomy bag etc. This embodiment provides the advantage of providing anti-inflammatory, anti-bacterial, and anti-viral effect, which advantage is especially important in the early stages of ostomy.

In another embodiment according to the present invention NO eluting poymer is applied on a intravenous dressing. When the device according to the present invention is in form of a intravenous dressing, the area surrounding an intravenous catheter is provided with anti-microbial, anti-inflammatory, anti-thrombotic and anti-viral effect. This embodiment has the advantage of protecting an area which otherwise is exposed to a high possibility of getting in contact with infectious material.

The device according to the present invention elutes nitric oxide (NO) from said eluting polymer in a therapeutic dose in the oral cavity, such as between 1 to 100 ppm, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 91, 92, 93, 94, 95, 96, 97, 98, 99, or 100 ppm.

In the embodiments of the present invention it may be suitable to control or regulate the time span of NO release from the device according to the invention. This may be accomplished by integrating other polymers or materials in said device. These polymers or materials may be chosen from any suitable material or polymer, such as polyvinylacetates, polylacticacids, starch, cellulose, polyhydroxyalkanoates, polyesters, polycaprolactone, polyvinylalcohol, protein based plastics, gelatine, biogradable polymers, and other soluble plastics.

The NO-eluting polymers in the devices according to the present invention may be combined with silver, such as hydroactivated silver. The integration of silver in the devices according to the present invention gives the healing process an extra boost. Preferably the silver is releasable from the devices in the form of silver ions.

The device according to the present invention may be manufactured by, for example electro spinning of L-PEI or other polymers comprising L-PEI or being arranged in combination with L-PEI. L-PEI is the charged at a characteristic voltage, and a fine jet of L-PEI releases as a bundle of L-PEI polymer fibres. This jet of polymer fibres may be directed to a surface to be treated. The surface to be treated may for example be any suitable material in respect of a device according to the present invention. The electro spun fibres of L-PEI then attach on said material and form a coating/layer of L-PEI on the device according to the invention.

It is of course possible to electro spin the other NO-eluting polymers, according to above, on the device according to the invention while still being inside the scope of the present invention.

In one embodiment the NO-eluting polymers according to the present invention are electro spun in such way that pure NO-eluting polymer fibres may be obtained.

It is also within the scope of the present invention to electro spin a NO-eluting polymer together with other suitable polymer/polymers.

Gas stream spinning, or air spinning, of said NO-eluting polymers onto the device according to the present invention is also within the scope of the present invention.

The manufacturing process according to the present invention presents the advantages of large contact surface of the NO-eluting polymer fibres with the area to be treated, effective use of NO-eluting polymer, and a cost effective way of producing the device according to the present invention.

Hereinafter, some potential uses of the present invention are described:

A method of treating at least one wound, comprising
applying a device, that comprises a nitric oxide (NO) eluting polymer configured for eluting a therapeutic dosage of nitrogen oxide (NO) when used for said treatment, and thereby exposing said treatment site of said at least one wound on a body to said nitric oxide when said polymer in use elutes nitrogen oxide (NO) by eluting a therapeutic dose of nitric oxide from said nitric oxide eluting polymer to said treatment site.

The method according to above, wherein said site of said at least one wound is a head, face, neck, shoulder, back, arm, hand, stomach, genital, thigh, leg, or foot, of a body, and wherein said method comprises applying a condom/sheath, sock, patch/pad, cream, ointment, foam, hydrogel, and tape/coating to said head, face, neck, shoulder, back, arm, hand, stomach, genital, thigh, leg, or foot, of a body, for said exposure.

Use of nitric oxide (NO) in a therapeutic dose for therapeutically treating and/or preventing at least one wound on a body.

The invention may be implemented in any suitable form. The elements and components of the embodiments according to the invention may be physically, functionally, and logically implemented in any suitable way. Indeed, the functionality may be implemented in a single unit, in a plurality of units, or as part of other functional units.

Although the present invention has been described above with reference to specific embodiments, it is not intended to be limited to the specific form set forth herein. Rather, the invention is limited only by the accompanying claims and, other embodiments than the specific above are equally possible within the scope of these appended claims.

In the claims, the term "comprises/comprising" does not exclude the presence of other elements or steps. Furthermore, although individually listed, a plurality of means, elements or method steps may be implemented. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided merely as a clarifying example and shall not be construed as limiting the scope of the claims in any way.

## Claims

1. A wound care device configured for treatment of at least one wound on a mammal body,
wherein
said device comprises a nitric oxide (NO) eluting polymer configured for eluting a therapeutic dosage of nitrogen oxide (NO) when used for said treatment, and
wherein said device is configured for exposing a topical wound area of said wound on a body to said nitric oxide when said polymer in use elutes nitrogen oxide (NO).

2. Device according to claim 1, wherein said polymer is selected from the group consisting of polyalkyleneimines, S-nitrosylated polymer, and poly(alkylenimine)diazeniumdiolates, or any combinations thereof.

3. Device according to claim 1, wherein said polymer is L-PEI (linear polyethyleneimine), prior to use loaded with nitric oxide (NO), arranged for release of the nitric oxide (NO) at said treatment site of said at least one wound on a body.

4. Device according to claim 1, wherein the device has a form selected from the group consisting of a condom/sheath, a sock, a patch/pad/dressing, ostomy bag, gaskets for ostomy bags, and a tape/coating, adapted to be applied on or at said treatment site of said at least one wound on a body for treatment of wounds.

5. Device according to claim 4, wherein said condom/sheath, sock, patch/pad/dressing, ostomy bag, gaskets for ostomy bags, and tape/coating is a polyurethane or polyethylene condom/sheath, sock, patch/pad/dressing, ostomy bag, gaskets for ostomy bags, or tape/coating, including said nitric oxide (NO) eluting polymer configured for in use eluting said nitric oxide (NO) to said treatment site of said at least one wound on a body for treatment of said wounds.

6. Device according to any of claims 1 to 5, including a water bag or sealed water sponge, configured for releasing water there from, when activated, to said device, and wherein said polymer is activateable to elute nitric oxide (NO) upon contact with said water.

7. Device according to any preceding claim, wherein said device is partly disintegrable when subjected to moisture or water.

8. Device according to any preceding claim, wherein said polymer comprises silver, configured for therapeutic treatment of said site of said at least one wound on the body.

9. Device according to any preceding claim, wherein said polymer is comprised in the device in form of fibers, nano-particles or micro-spheres.

10. Device according to claim 9, wherein said fibres, nano-particles, or micro-spheres, are in the form of a gel, cream, foam, or hydrogel, or combinations thereof.

11. Device according to claim 9, wherein said nano-particles, or micro-spheres, are integrated with, preferably encapsulated in, a material, selected from the group consisting of polyvinylacetates, polylacticacids, starch, cellulose, polyhydroxyalkanoates, polyesters, polycaprolactone, polyvinylalcohol, protein based plastics, and/or gelatine, or combinations thereof.

12. Device according to any preceding claim, wherein said device comprises a compound comprising a drug and said nitric oxide (NO) eluting polymer, and wherein said device is configured for exposing said wound area to said drug and to said nitric oxide when said polymer in use elutes nitrogen oxide (NO), such that that wound area is more susceptible to said drug.

13. A device configured for therapeutic treatment, according to any of the preceding claims,
wherein said device comprises a compound comprising
a drug and a said nitric oxide (NO) eluting polymer, wherein said nitric oxide (NO) eluting polymer is configured for eluting a therapeutic dosage of nitrogen oxide (NO) when used for said therapeutic treatment,
wherein said device is configured for exposing a treatment site in or on a body to said drug and to said nitric oxide when said polymer in use elutes nitrogen oxide (NO), such that that treatment site is more susceptible to said drug.

14. A manufacturing process for a wound care device configured for treatment of at least one wound according to claim 1, comprising:
selecting a plurality of nitric oxide eluting polymeric particles, preferably nano fibres, nano particles or micro spheres, and
deploying said nitric oxide eluting particles into a suitable form, or as a coating onto a carrier, to form said device,
wherein said deploying comprises electro, air, or gas stream spinning of said particles.

15. Use of a nitric oxide (NO) eluting polymer for the manufacture of a device for the treatment of at least one wound,
wherein
nitric oxide is loaded to said device so said device elutes nitric oxide (NO) from said eluting polymer in a therapeutic dose when used at a site of at least one wound on a body.

16. Use according to claim 15, wherein said therapeutic dose is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 91, 92, 93, 94, 95, 96, 97, 98, 99, or 100 ppm.
